# FASCICULE DE BREVET EUROPEEN

(11) **EP 2 560 616 B1**
(45) Date de publication et mention de la délivrance du brevet: **19.07.2017**
(21) Numéro de dépôt: 11715556.4
(22) Date de dépôt: 21.04.2011
(51) Int. Cl.: A61K 9/107, A61P 27/04, A61K 31/717, A61K 31/728, A61K 31/737, A61K 47/36, A61K 47/38, A61K 33/14, A61K 31/74, A61K 31/727, A61K 9/00

(54) **EMULSION LACRYMIMETIQUE**
KÜNSTLICHE TRÄNENEMULSION
ARTIFICIAL TEAR EMULSION

(30) Priorité: 21.04.2010 FR 1053030
(43) Date de publication de la demande: 27.02.2013
(73) Titulaire: Horus Pharma, 06700 Saint-Laurent Du Var (FR)
(72) Inventeur: CLARET, Claude, F-06100 Nice (FR); CLARET, Martine, F-06100 Nice (FR); GARD, Carole, Villa Zellidja 06110 Le Cannet (FR); LAMPRECHT-WEISSENBORN, Nicola, F-25000 Besançon (FR)
(74) Mandataire: Regimbeau
(86) Numéro de dépôt international: PCT/EP2011/056447
(87) Numéro de publication internationale: WO 2011/131765

(56) Documents cités:
- EP-A1- 2 377 508
- WO-A1-95/05163
- WO-A1-2007/085327
- WO-A1-2009/063962
- WO-A2-2011/115700
- DE-A1- 19 527 132
- FR-A1- 2 921 253
- GB-A- 2 464 430
- DATABASE CA, [Online] 10 février 2009 (2009-02-10), PAOLONE J F: "Composition of active principles for hydration, cellular regeneration, scavenging of free radicals and dermal protection", XP002619056, extrait de CA Database accession no. 153-589361
- "2.2.8 Viscosity" In: anonymous: "European Pharmacopoeia 7.0", 1 January 2008 (2008-01-01) pages 27-30,
- GUPTA SYAMASRI: "Biocompatible microemulsion systems for drug encapsulation and delivery", CURRENT SCIENCE (BANGALORE), vol. 101, no. 2, July 2011 (2011-07), pages 174-188, ISSN: 0011-3891(print)

## Description

La présente invention concerne des émulsions lipidiques, en particulier lacrymimétiques, comprenant un polymère mucomimétique, leur utilisation et un procédé permettant de les obtenir. La présente invention porte plus spécifiquement sur l'utilisation de ces compositions dans le domaine ophtalmique. Ces compositions peuvent plus particulièrement être destinées à la prévention ou au traitement de la sécheresse oculaire et/ou de troubles liés à cette sécheresse oculaire. Ces compositions peuvent encore être destinées au transport de substances actives, en particulier dans l'oeil.

La surface oculaire est une muqueuse de transition entre le milieu extérieur et les structures intraoculaires. Les paupières constituent la première ligne de défense de la surface oculaire et participent à l'entretien du film lacrymal qui recouvre l'épithélium conjonctival, notamment grâce aux glandes lacrymales et aux glandes de Meibomius situées respectivement dans les paupières inférieures et supérieures.

Ce film lacrymal est un film fluide continu présentant une épaisseur comprise entre 3 et 10 µm et un volume d'environ 10 µl. Ce film est continuellement renouvelé, à raison d'environ 1 µl/min.

Parmi les fonctions du film lacrymal on peut citer conserver la surface oculaire humide, produire une surface lisse permettant de voir sans distorsion, protéger la cornée et l'épithélium conjonctif des agressions extérieures (matières irritantes, poussières, bactéries...), et transporter des substances biologiques utiles pour la physiologie de l'oeil, dont l'oxygène pour la cornée.

Par ailleurs, le film lacrymal est composé de trois couches, lipidique, aqueuse et mucinique :
- la couche lipidique, d'épaisseur 100 nm environ, sécrétée par les glandes de Meibomius, qui permet notamment de réduire l'évaporation et de stabiliser le film lacrymal ;
- la couche aqueuse, qui permet de nourrir, d'hydrater et de protéger l'épithélium cornéo-conjonctival ; outre sa forte teneur en eau, elle est riche en éléments nutritifs, enzymes, agents antibactériens, facteurs de cicatrisation et renferme un fort gradient de concentration de mucine ; et
- la couche mucinique, qui permet l'ancrage du film lacrymal.

La structure en trois couches du film lacrymal peut encore être présentée comme un système comportant deux phases principales :
- une phase hydrophobe, non-polaire, se trouvant au contact de l'air et composée de phospholipides, esters, triglycérides et acides gras libres, et
- une phase hydrophile, polaire, anionique, composée de glycoprotéines, lipides, électrolytes, enzymes, polysaccharides et d'eau.

L'altération d'une de ces couches peut être à l'origine d'un syndrome de sécheresse oculaire, par exemple en raison d'une hyposécrétion des glandes lacrymales ou d'un dysfonctionnement des glandes de Meibomius, donc de la couche lipidique du film lacrymal, et entraîner une hyper-évaporation.

Parmi les symptômes de la sécheresse oculaire on peut citer douleur, démangeaison, sensation de corps étranger, brûlure, photophobie et inconfort général. Ainsi, cette maladie est invalidante et perturbe la qualité de vie des patients.

Par ailleurs, la sécheresse oculaire, quelle que soit son étiologie, peut être à l'origine d'une kératite et/ou d'une conjonctivite.

Ce syndrome de l'oeil sec ou de la sécheresse oculaire est assez fréquent dans la population, puisqu'il est estimé qu'environ 15 à 20% de la population âgée de plus de 65 ans en souffre. Les formes modérées et sévères, impliquant une kératite ou kératoconjonctivite sèche, représenteraient 25 à 30% des cas.

La première ligne de traitement des syndromes secs, indépendamment de la sévérité, comprend les substituts lacrymaux, également appelées larmes artificielles, qui agissent en premier lieu sur les symptômes.

Historiquement, les substituts lacrymaux simples à base de chlorure de sodium ont été progressivement remplacés par des solutions à base de dérivés cellulosiques ou de carbomères, permettant d'augmenter la viscosité des solutions et d'obtenir une action plus rémanente.

Cependant, encore plus récemment deux grandes catégories présentent semble-t-il une plus grande efficacité :
- les émulsions lipidiques, et
- les solutions à base de hyaluronate de sodium. DE-A-19527132 divulgue une composition ophtalmique contenant une émulsion de type huile dans eau comprenant un phospholipide et un lipide autre que le phospholipide, et de l'hydroxypropylméthylcellulose. WO-A-9505163 divulgue des émulsions bioadhésives du type lipide dans l'eau contenant des macromolécules mucoadhésives. Malheureusement, la grande majorité de ces solutions ne s'adressent qu'à la restauration partielle d'une des composantes du film lacrymal.

En particulier, les émulsions lipidiques, hydrophobes, agissent sur la phase hydrophobe ou lipidique du film lacrymal en limitant le phénomène d'hyper-évaporation, tandis que les solutions à base d'acide hyaluronique, mucomimétique, agissent sur la phase hydrophile du film lacrymal et favorisent l'ancrage du film lacrymal.

D'autre part, ces produits devant être stériles, une grande partie des solutions existantes comprennent des conservateurs ayant pour objet de détruire les microorganismes après ouverture et pendant l'utilisation du produit.

Ces conservateurs peuvent notamment être :
- toxiques ou irritants pour les cellules de la cornée ou la conjonctive, les cellules à mucus (qui libèrent des mucines essentiels à la stabilité du film lacrymal) ou les cellules épithéliales de recouvrement, et/ou
- source d'allergie, d'inflammation, en particulier pour les pathologies chroniques comme le glaucome, l'allergie ou la sécheresse oculaire, qui nécessitent des instillations fréquentes de produits contenant des conservateurs.

Les conséquences cliniques peuvent ainsi être une aggravation des symptômes et/ou une évolution vers une maladie chronique.

Très fréquemment, les solutions contiennent des agents appelés tensioactifs afin de conférer une bonne stabilité physico-chimique à la composition. Ces tensioactifs peuvent présenter des effets délétères voisins des agents conservateurs soit un caractère irritant, allergisant, voire cytotoxique. En particulier, ces agents, également appelés émulsifiants, surfactants ou agents mouillants, présents en faible quantité, sont utilisés pour stabiliser les gouttelettes d'huiles dans les solutions à base d'huile ou émulsions lipidiques, commercialisées avec l'indication de sécheresse oculaire par hyper-évaporation.

Ainsi, les compositions existantes peuvent être insuffisamment efficaces, voire présenter des effets indésirables, comme la destruction de cellules saines, une réactivation des causes de la sécheresse oculaire par déstabilisation de la couche lipidique du film lacrymal ou des réactions allergiques. Elles peuvent encore être instables, nécessiter des conditions particulières de conservation (par exemple au froid....), et/ou nécessiter une utilisation très fréquente, en raison d'une durée d'action insatisfaisante.

La présente invention vise donc à résoudre en tout ou partie les problèmes évoqués ci-dessus. En particulier, l'invention vise à fournir une composition, notamment une émulsion, ayant une efficacité améliorée, dépourvue d'effets indésirables, ou tout au moins limitant ceux-ci au maximum, une stabilité améliorée, facile à obtenir, comprenant et/ou utilisant lors de son obtention un minimum de produits délétères, allergisants et/ou toxiques, et/ou ayant une action reconstituante sur l'ensemble du film lacrymal.

Selon un premier aspect, la présente invention a pour objet une émulsion de type huile dans eau dépourvue de conservateurs, dont les globules présentent une taille maximale inférieure ou égale à 220 nm, ayant une osmolarité allant de 110 et 180 mOsm/l, comprenant:
- au moins un polymère mucomimétique choisi parmi les acides hyaluroniques, le sulfate de dextrane et le sulfate de chondroïtine,
- au moins un lipide de type phospholipide choisi parmi les lécithines,
- au moins un lipide autre que phospholipide,
- au moins un polymère stabilisant choisi parmi les polymères de cellulose,
- milieu tampon citrate, de l'eau, pour son utilisation dans le traitement ou la prévention des affections ophtalmiques.
- L'émulsion peut comprendre: éventuellement du chlorure de sodium, et
- éventuellement :
   ∘ une substance active et/ou
   ∘ une substance participant à la restauration de la composition de la couche aqueuse du film lacrymal ou produits de suppléance lacrymale.

On entend par « au moins un », dans la présente description, un ou plusieurs, et en particulier un.

L'émulsion selon l'invention est une émulsion de type huile dans l'eau dont la phase aqueuse comprend au moins un polymère stabilisant et au moins un polymère mucomimétique, et la phase huileuse comprend au moins un lipide de type phospholipide et au moins un lipide autre que phospholipide. Un polymère mucomimétique peut être un polymère de type polyoside, en particulier choisi parmi les polysaccharides, encore appelés glycanes ou polyholosides, et plus particulièrement parmi les glycosaminoglycanes (GAG) et les acides hyaluroniques. Ces polymères, également désignés sous le terme mucopolysaccharides acides, peuvent être caractérisés par une forte capacité de rétention de l'eau leur conférant des propriétés mucomimétiques.

En particulier, les polysaccharides comprennent au moins 5, notamment au moins 10, en particulier au moins 20 unités osidiques ou monosacharidiques.

Parmi les polysaccharides, on peut citer :
- sulfate de dextrane, qui est un polysaccharide complexe, en particulier de poids moléculaire allant de 4 à 500 kDa,
- arabinogalactane, qui est un biopolymère constitué de monosaccharides d'arabinose et de galactose, constituant naturel de certaines gommes et des parois de certaines cellules mycobactériennes,
- héparine, en particulier de poids moléculaire allant de 6 à 30 kDa,
- sulfate de kératane,
- sulfate de chondroïtine, en particulier de poids moléculaire environ 50 kDa
- sulfate de dermatane, et
- acide hyaluronique, qui est un polymère de disaccharide de haute viscosité, présent naturellement dans de nombreux tissus, dont les tissus conjonctifs et l'un des principaux constituants de la matrice extracellulaire.

Ce dernier peut être obtenu par extraction depuis les tissus animaux ou par fermentation bactérienne.

Le polymère mucomimétique peut être plus préférentiellement choisi parmi les glycosaminoglycanes et les acides hyaluroniques et leurs mélanges. Selon l'invention, le polymère mucomimétique est choisi parmi les acides hyaluroniques, le sulfate de dextrane et le sulfate de chondroïtine. Le polymère mucomimétique peut avoir un poids moléculaire allant de 10 à 10 000 kDa, notamment de 500 à 1 500 kDa, voire d'environ 1 000 kDa.

En particulier, l'émulsion comprend une teneur en polymère mucomimétique allant de 0,01 à 5 % en poids par rapport au poids total de l'émulsion, notamment allant de 0,05 à 2,5 % en poids, en particulier allant de 0,1 à 1 % en poids, voire allant de 0,15 à 0,5 % en poids, et encore plus particulièrement d'environ 0,2 % en poids.

Les lipides de type phospholipide, plus communément appelés phospholipides, sont des lipides comprenant un groupe phosphate, c'est à dire un assemblage de 2 acides gras, de glycérol et de phosphate.

Ils peuvent comprendre de la sphingosine (sérine+acide gras), un acide gras, un phosphate et un alcool azoté ou bien deux acides gras, une molécule de glycérol, un phosphate et un alcool azoté.

En particulier, le phospholipide peut être choisi parmi les phosphoacylglycérides, encore appelés phosphacylglycérols, les phosphosphingolipides, les phosphonoshingolipides les phosphoglycolipides et les phosphosacharolipides. Selon l'invention, le phospholipide est choisi parmi les lécithines, encore appelées phosphatidylcholines, extraites à partir de soja, d'oeuf ou de tournesol. Les lécithines sont de préférence extraites à partir de soja.

La lécithine peut être utilisée seules ou en combinaison. En particulier plusieurs lécithines différentes sont présentes dans l'émulsion.

L'émulsion peut comprendre une teneur en phospholipide allant de 0,01 à 3 % en poids par rapport au poids total de l'émulsion, notamment allant de 0,02 à 1 % en poids, en particulier allant de 0,05 à 0,5 % en poids, voire allant de 0,075 à 0,2 % en poids, et encore plus particulièrement d'environ 0,1 % en poids.

L'émulsion comprend au moins un lipide autre que phospholipide, en particulier un lipide de type glycéride, encore appelé glycéride.

Le lipide de type glycéride peut être un lipide de la classe des acylglycérol ou glycéride, en particulier choisi parmi les huiles de ricin, de soja, de sésame, de paraffine, de lanoline, de vaseline, de mais, de glycérine, ou de monoglycéride, de triglycérides.

Tout particulièrement ledit glycéride est un triglycéride, préférentiellement un triglycéride à chaîne moyenne, dont les 3 groupements hydroxyle du glycérol sont estérifiés par des acides gras, comportant de 4 à 22 atomes de carbone, en particulier comportant 8 à 12 atomes de carbone, de préférence choisis parmi l'acide caprique, l'acide caprylique et leurs mélanges.

L'émulsion peut comprendre une teneur en lipide autre que phospholipide allant de 0,01 à 5 % en poids par rapport au poids total de l'émulsion, notamment allant de 0,05 à 2,5 %, en particulier allant de 0,1 à 1 % en poids, voire allant de 0,15 à 0,5 % en poids, et encore plus particulièrement d'environ 0,2 % en poids.

Tout particulièrement, l'émulsion comprend un rapport pondéral lipide autre que phospholipide / phospholipide allant de 0,1 à 10, notamment allant de 0,2 à 5, en particulier de 0,5 à 2, voire d'environ 1.

L'émulsion comprend au moins un polymère stabilisant, en particulier un polymère ionique, plus particulièrement un polymère anionique. Selon l'invention, le polymère stabilisant est choisi parmi les polymères de cellulose. Le polymère stabilisant peut être choisi parmi les polymères d'alcool de polyvinyle, les polysorbates, et de type cellulose, notamment la méthylcellulose, l'éthylcellulose, l'hydroxypropylcellulose, la carboxyméthylcellulose. En particulier, le polymère est du carboxyméthylcellulose.

Le polymère stabilisant est en particulier en polymère sodique, notamment de la carboxyméthylcellulose sodique.

La carboxyméthylcellulose sodique peut présenter un formule générale [C₆H₇O₂(OH)ₓ(OCH₂COONa)_{y}]ₙ avec y, degré de substitution, permettant une solubilisation dans l'eau, compris entre 0,6 et 1, x compris entre 1 et 2,4 (respectant x+y=3) et n compris entre 80 et 1500.

Les formes solubles peuvent présenter une viscosité allant de 5 à 2000 cps en solution de 1 % en poids, notamment de 1000 à 2000 cps, voire d'environ 1500 cps. La carboxyméthylcellulose peut présenter une teneur en sel rapportée à la substance sèche d'au moins 6,5% et au plus de 10,8% de sorte à correspondre à l'une des spécifications de la pharmacopée européenne.

Le polymère stabilisant, notamment ionique, peut présenter un poids moléculaire allant de 17 000 à 300 000 g/mol.

La composition peut comprendre une teneur en polymère stabilisant, en particulier ionique, allant de 0,01 à 5 % en poids, notamment allant de 0,05 à 2,5 %, en particulier allant de 0,1 à 1 % en poids, voire allant de 0,15 à 0,5 % en poids, et encore plus particulièrement d'environ 0,2 % en poids par rapport au poids total de la composition.

La présence du polymère stabilisant, notamment ionique, et en particulier du carboxyméthylcellulose, notamment sodique, permet à l'émulsion comprenant au moins un phospholipide et un polymère mucomimétique de présenter une stabilité satisfaisante. Cette stabilité peut être constatée notamment par la taille des globules de la phase huileuse qui reste sensiblement stable, notamment une augmentation de taille de moins de 10 %, après 3 semaines.

L'émulsion peut présenter un phénomène de coalescence, cependant une simple agitation peut permettre de redisperser les globules.

L'émulsion selon l'invention peut présenter un pH allant de 6 à 8, notamment allant de 6,7 à 7,7, en particulier allant de 7,0 à 7,6, voire d'environ 7,4.

L'émulsion peut tout particulièrement présenter un pH allant de 5,5 à 8, notamment de 6 à 7,7, en particulier allant de 6,2 à 7,5, voire d'environ 7,0.

Tout particulièrement, l'émulsion selon l'invention comprend un milieu tampon, ou tampon. Un tampon peut être un tampon phosphate, acétate, citrate. Selon l'invention, il s'agit d'un tampon citrate.

Tout particulièrement le tampon citrate peut permettre d'améliorer la stabilité de l'émulsion.

L'émulsion selon l'invention comprend donc avantageusement du citrate en quantité appropriée pour ajuster le pH à la valeur souhaitée.

L'émulsion est de type huile dans eau, la partie huile se présentant sous la forme de globules comprenant les constituants lipidiques définis précédemment et les éventuels autres constituants hydrophobes et/ou lipophiles de l'émulsion.

La petite taille de ces globules peut participer à la bonne stabilité de l'émulsion.

Pour la préparation d'une émulsion sans conservateurs, telle que définie ci-après, on cherchera de préférence à ce que 98 à 99% de l'émulsion doit pouvoir passer par un filtre de 0,22 µm, taille d'un filtre généralement employé pour la stérilisation de l'émulsion. Des globules d'une taille supérieure à 220 nm peuvent passer à travers un filtre de 0,22 µm en modifiant temporairement leur forme, cependant ralentissant le procédé de filtration.

Les globules présentent une taille maximale inférieure ou égale à 220 nm, en particulier inférieure ou égale à 160 nm.

L'émulsion selon l'invention est particulièrement adaptée pour une utilisation topique dans le traitement ou la prévention des affections ophtalmiques, notamment comme collyre, permettant une tolérance améliorée du fait que l'émulsion est stable sans l'aide d'agent tensioactif, ou du moins dans une proportion suffisamment faible pour être tolérée par la surface oculaire.

Selon un mode préféré de réalisation, la présente émulsion est dépourvue de conservateurs, notamment chimiques, utilisés seuls ou en association. Lesdits conservateurs peuvent en particulier être choisis parmi la liste des produits autorisés par la réglementation, comme :
- les ammoniums quaternaires, notamment chlorure de benzalkonium, alkyldiméthylbenzylammonium, cétrimide, chlorure de cétylpyridinium, bromure de benzododécinium, chlorure de benzothonium, chlorure de cetalkonium,
- les conservateurs mercuriels, comme nitrate/acétate/borate phénylmercurique, thiomersal,
- les conservateurs alcooliques, comme chlorobutanol, alcool benzylique, phényléthanol, alcool phénylethylique,
- les acides carboxyliques, tels qu'acide sorbique,
- les phénols, en particulier méthyl/propyl parabène,
- les amidines, par exemple chlorhexidine digluconate, et/ou
- l'EDTA, agent chélateur potentialisant l'efficacité des conservateurs, en association avec au moins un conservateur.

En particulier l'émulsion est préférentiellement dépourvue d'EDTA en tant que tel.

Par « dépourvue de conservateur » ou « sans conservateurs » on entend au sens de la présente invention une teneur en conservateurs et/ou en EDTA inférieure ou égale à 10 ppm, notamment inférieure ou égale à 1 ppm, voire égale à 0 ppm.

Selon un autre aspect de la description, la composition peut comprendre au moins un agent conservateur, mais de préférence à faible concentration, par exemple à une concentration inférieure ou égale à 0,1 % en poids, notamment inférieure ou égale à 0,05 % en poids, voire inférieure à 0,01 % en poids par rapport au poids total de l'émulsion.

L'agent conservateur est en particulier présent dans la phase aqueuse.

Selon un mode avantageux de réalisation de l'invention, l'émulsion est dépourvue d'agent tensioactifs, utilisés seuls ou en associations. Lesdits tensioactifs sont des agents tensioactifs usuels pour la préparation d'émulsions, en particulier dans le domaine de la cosmétique et de la pharmacie, en particulier choisis parmi la liste des produits autorisés par la réglementation, comme :
- les polysorbates,
- les polyéthylènes glycol et leurs dérivés,
- le polyoxyéthylène-40-stéarate,
- les esters de sorbitane,
- les copolymères polyoxyéthylène-polyoxypropylène,
- les alcools polyvinyliques, et
- les polymères de polyvinylpirrolidone

Par « dépourvue d'agent tensioactif » ou « sans agents tensioactifs », on entend au sens de la présente invention une teneur inférieure ou égale à 0,1% en poids par rapport au poids total de l'émulsion, notamment inférieure à 0,01% en poids, voire égale à 0% en poids.

Les phospholipides entrant dans la composition de l'émulsion selon l'invention tels que définis précédemment, et en particulier les lécithines, ne sont pas des agents tensioactifs usuels employés comme agents tensioactifs dans la préparation d'émulsions dans le domaine de la cosmétique ou de la pharmacie. En tout état de cause, ils ne sont bien entendu pas exclus de la composition de l'émulsion selon l'invention.

Selon un autre mode de réalisation de l'invention, l'émulsion peut comprendre au moins un agent tensioactif, de préférence à faible concentration, c'est-à-dire à une concentration inférieure ou égale à 0,5 % en poids par rapport au poids total de l'émulsion, notamment inférieure ou égale à 0,25 % en poids, voire inférieure à 0,15 % en poids.

Selon un mode particulièrement préféré de réalisation de l'invention, l'émulsion est dépourvue d'agent tensioactif et de conservateur.

Ceci peut tout particulièrement permettre de limiter, diminuer, voire éviter, des effets secondaires indésirables, notamment irritation(s), sur l'oeil et/ou les muqueuses, en particulier proche de l'oeil.

De manière avantageuse, l'émulsion selon l'invention présente une faible viscosité proche de celle de l'eau, notamment une viscosité dynamique inférieure ou égale à 10⁻¹ Pa.s, en particulier allant de 1,5.10⁻³ à 8.10⁻², et tout particulièrement allant de 3.10⁻³ à 6.10⁻² Pa.s.

L'émulsion selon l'invention présente avantageusement une limpidité telle que définie par European Pharmacopeia 7.0 allant de 0 à 5000 NTU, en particulier allant de 0 à 1000 NTU, tout particulièrement d'environ 200 à 700 NTU, voire d'environ 500 NTU.

L'émulsion selon l'invention est particulièrement appropriée pour une utilisation sous forme de collyre, de préférence de faible viscosité telle que définie ci-dessus et avantageusement limpide.

L'émulsion selon l'invention particulièrement adaptée pour une utilisation topique dans le traitement ou la prévention des affections ophtalmiques, en particulier comme collyre, peut avoir des propriétés lacrymimétiques, c'est à dire à présenter, une fois déposée, une structure proche ou identique à celle du liquide lacrymal. Selon l'invention, l'émulsion est hypo-osmolaire et a une osmolarité allant de 110 à 180 mosmol/l tout particulièrement de 120 à 150 mosmol/l voire être d'environ 135 mosmol/l.

Une osmolarité hypo-osmolaire peut permettre de compenser l'hyperosmolarité généralement constatée chez les patients atteints de sécheresse oculaire.

L'homme du métier sait ajuster l'osmolarité de l'émulsion selon l'invention par l'ajout d'une quantité appropriée de sel, en particulier avec du chlorure de sodium, du chlorure de potassium ou du bicarbonate de sodium et/ou de potassium, préférentiellement du chlorure de sodium.

La teneur en eau dépend de la teneur des autres constituants de l'émulsion selon l'invention. De manière préférentielle, en particulier pour une émulsion adaptée à une utilisation topique dans le traitement ou la prévention des affections ophtalmiques, en particulier sous forme de collyre, l'émulsion comprend une teneur en eau supérieure ou égale à 90 % en poids par rapport au poids total de l'émulsion, notamment supérieure à 95 % en poids, en particulier supérieure à 98 % en poids, et tout particulièrement supérieure à 99 % en poids.

A cette formulation, il est possible d'ajouter des substances, en particulier dans la phase aqueuse, participant à la restauration de la composition de la couche aqueuse du film lacrymal ou produits de suppléance lacrymale, comme :
- vitamine A,
- vitamine E,
- vitamine B12,
- albumine,
- ions sodium, potassium, calcium, chlorure et bicarbonate, et
- solution tampon.

La composition ou l'émulsion peut comprendre un ou plusieurs agent(s) actif(s), notamment un agent thérapeutiquement actif. Cet agent actif est en particulier destiné à agir au niveau de l'oeil ou est à destination ophtalmique.

Cette composition ou émulsion ophtalmique peut être utilisée à la préparation d'un produit destiné au traitement d'affections ophtalmiques en association avec la restauration du film lacrymal dans les syndromes secs, en particulier en y incorporant un principe actif doté d'un effet thérapeutique.

L'agent actif, notamment à usage ophtalmique, peut-être choisi parmi les antiseptiques, antibiotiques, anti-viraux, anti-inflammatoires, anti-allergiques, anti-glaucomateux, anti-sécheresse, vaso-constricteurs, les anesthésiques, les mydriatiques, les myotiques, les produits de diagnostic et les produits pour le traitement des affections rétiniennes.

Parmi les principes actifs à usage ophtalmique, on peut citer :
- les anesthésiques, comme Procaine, Chloroprocaine, Lidocaine, Mepivacaine, Tetracaine, Proparacaine,
- les anti-inflammatoires stéroïdiens, comme Bethamethasone, Dexamethasone, Fluorometholone, Loteprednol Etabonate, Medrysone, Prednisolone, Rimexolone, Methylprednisolone, Prednisone, Fluocinolone, Triamcinolone acetonide,
- les anti-inflammatoires non-stéroïdiens, comme Flurbiprofen, Suprofen, Diclofenac, Ketorolac, Aspirin, Indomethacin, Ibuprofen, Naproxen,
- les immunomodulateurs, comme Cyclosporine, Azathioprine, Bromocriptine, Methotrexate, Dapsone, Cyclophosphamide, Chlorambucil, Colchicine,
- les anti-glaucomateux, comme Dipivefrin, Epinephryl, Apraclonidine, Brimonidine, Betaxolol, Carteolol, Levobunolol, Metipranolol, Timolol, Carbachol, Pilocarpine, Physostigmine, Echothiophate, Acetazolamide, Brinzolamide, Dorzolamide, Methazolamide, Latanoprost, Bimatoprost, Travoprost, Unoprostone,
- les antibiotiques et anti-infectieux, comme Cefotaxime, Ceftazidime, Cefuroxime, Cefazoline, Cephalothin, Ampicillin, Oxacillin, Ticarcillin, Sodium sulfacetamide, Sulfisoxazole, Sulfamethoxazole, Neomycin, Gentamicin, Tobramycin, Amikacin, Norfloxacin, Ciprofloxacin, Ofloxacin, Gatifloxacin, Levofloxacin, Moxifloxacin, Bacitracin, Gramicidin, Polymyxin B, Erythromycin, Chloramphenicol, Trimethoprim, Oxytetracycline, Vancomycin, et
- les antiviraux, comme Cidofovir, Formivirsen, Foscarnet, Ganciclovir, Valganciclovir, Trifluridine, Acyclovir, les antifongiques, Natamycin, Itraconazole, Ketoconazole, Miconazole, Amphotericin B, Fluconazole, Flucytosine.

Cependant, d'autres principes actifs et d'autres classes thérapeutiques pourront être envisagés.

Selon un aspect particulier de la description, la composition comprend essentiellement :
- du polymère mucomimétique, en particulier de l'acide hyaluronique, notamment à une teneur d'environ 0,2 % en poids par rapport au poids total de la composition,
- de la phosphatidylcholine, en particulier à une teneur d'environ 0,1 % en poids par rapport au poids total de la composition,
- au moins un triglycéride, en particulier d'acide caprique et/ou caprylique, notamment à une teneur d'environ 0,1 % en poids par rapport au poids total de la composition,
- du polymère stabilisant, en particulier de la carboxyméthylcellulose, notamment à une teneur d'environ 0,1 % en poids par rapport au poids total de la composition,
- du citrate et/ou de l'acide citrique, notamment afin d'ajuster le pH à environ 7,
- du chlorure de sodium, notamment afin d'ajuster l'osmolarité, en particulier à 180 mosmol/l et
- de l'eau.

Tout particulièrement, la présente émulsion est stérilisée sans intervention d'agent(s) chimique(s), notamment utilisé(s) en tant que conservateur(s). Cette émulsion peut en particulier être stérilisée par filtration, en particulier via une filtration à 0,22 µm.

L'émulsion, la composition ou le médicament peut être conditionnée par remplissage aseptique en milieu stérile, dans des flacons de type unidose ou monodose, dans des flacons de type multidose permettant de protéger la solution de toute contamination pendant l'utilisation, notamment tels que décrits dans les documents suivants WO 2008/015505, US 2009/0294347, US 2007/0210121, EP 2 085 068, FR 2 816 600, US 5,232,687 ou FR 2 873 358, ou dans tout autre flacon destiné à l'administration topique de médicaments permettant d'éviter l'usage de conservateurs.

Selon un de ses aspects, la description concerne un ensemble comprenant un contenant tel que décrit ci-dessus et une émulsion selon l'invention.

Selon un mode de réalisation particulier l'émulsion dépourvue de conservateur présente dans l'ensemble est stérile et peut rester stérile, par exemple pendant au moins trois mois, notamment au moins six mois, en particulier au moins un an, voire au moins trois an, avant ouverture.

Une fois le flacon ouvert, et selon le type de flacon, l'émulsion peut être utilisé durant 1 mois, voire 3 mois.

Selon un autre de ses aspects, l'invention a pour objet une composition pharmaceutique ou un médicament comprenant, ou consistant en, une émulsion telle que définie ci-dessus, en particulier destiné à traiter ou à prévenir la sécheresse oculaire, les allergies et/ou les inflammations au niveau de l'oeil, en particulier liées à la sécheresse oculaire.

Selon un autre de ses aspects, l'invention a pour objet l'utilisation d'une composition selon l'invention pour la préparation d'une composition pharmaceutique ou d'un médicament, en particulier destiné à traiter ou à prévenir la sécheresse oculaire, les allergies et/ou les inflammations au niveau de l'oeil, en particulier lié à la sécheresse oculaire.

Selon encore un autre de ses aspects, l'invention a pour objet une émulsion ou d'une composition telle que définie ci-dessus comprenant en outre un composé actif, en particulier destiné à agir au niveau de l'oeil.

Selon encore un autre de ses aspects, l'invention a pour objet l'utilisation d'une composition ou d'une émulsion selon l'invention en tant que véhicule pour un composé actif, en particulier destinée à agir au niveau de l'oeil.

Lorsque cette émulsion est utilisée en tant que vecteur, elle peut permettre de diminuer la concentration de principe actif ou la posologie, le nombre d'applications et/ou le temps entre deux applications.

Sans vouloir être lié par cette théorie, ceci peut provenir du fait que ladite émulsion, peut être du fait de sa viscosité et des ses propriétés mucoadhésives, améliore le temps de résidence, et ainsi augmente la proportion de principe actif qui peut être délivré à la cible.

Selon encore un autre de ses aspects, l'invention a également pour objet l'utilisation d'une émulsion ou d'une composition selon l'invention en tant qu'agent pour restaurer et/ou remplacer le film lacrymal.

L'invention concerne en particulier une émulsion telle que définie ci-dessus et dans les exemples, pour son utilisation dans le traitement ou la prévention des affections ophtalmiques, plus particulièrement dans le traitement ou la prévention de la sécheresse oculaire, les allergies et/ou les inflammations au niveau de l'oeil. La description concerne une méthode pour le traitement ou la prévention de la sécheresse oculaire, les allergies et/ou les inflammations au niveau de l'oeil chez un sujet nécessitant un tel traitement, qui consiste à appliquer sur l'oeil ou les deux yeux dudit sujet, une quantité appropriée de l'émulsion selon l'invention telle que définie ci-dessus et dans les exemples.

Cette émulsion est avantageusement appliquée sous forme de gouttes que l'on laisse tomber au contact de l'oeil, selon les méthodes usuelles d'applications de compositions ophtalmiques, plus particulièrement de collyres. Le nombre de gouttes appliquées pour chaque application n'est généralement pas limitant dans la mesure où un excès d'émulsion sera éliminé par le battement naturel des paupières sur l'oeil. Le nombre d'applications quotidiennes dépendra des besoins propres à chaque sujet.

Toutefois, dans la mesure où la composition comprendrait un actif tel que défini précédemment, le nombre de gouttes appliquées et le nombre d'applications quotidiennes dépendra de l'actif qui sera délivré avec l'émulsion selon l'invention.

Selon un autre de ses aspects, la description a encore pour objet l'utilisation d'un polymère stabilisant, éventuellement en association avec un phospholipide, en tant qu'agent de stabilisation d'une émulsion comprenant un lipide s'adressant à la phase hydrophobe, un polysaccharide de propriété mucomimétiques ou mucoadhésives s'adressant à la phase hydrophile, associés dans une phase aqueuse à l'aide d'au moins un phospholipide et au moins un polymère ionique.

Selon encore un autre de ses aspects, la description a encore pour objet l'utilisation d'un polymère stabilisant, éventuellement en association avec un phospholipide, en tant qu'agent de stabilisation d'une émulsion comprenant :
- un lipide autre que le phospholipide dans la phase hydrophobe,
- un polymère mucomimétiques dans la phase aqueuse, en particulier en une teneur importante, notamment telle que définie ci-dessus.

Selon un autre de ses aspects, l'invention a pour objet un procédé de préparation d'une émulsion huile dans eau telle que définie selon l'une quelconque des revendications 1-11, comprenant les étapes suivantes :
a) mélanger un phospholipide choisi parmi les lécithines et un lipide autre que le phospholipide, notamment à une température supérieure à 30°C,
b) dissoudre un polymère mucomimétique choisi parmi les acides hyaluroniques, le sulfate de dextrane et le sulfate de chondroïtine dans un tampon, notamment citrate,
c) dissoudre un polymère stabilisant choisi parmi les polymères de cellulose dans la phase aqueuse de l'étape b),
d) disperser la phase huileuse de l'étape a) dans la phase aqueuse de l'étape c) par agitation,
e) contrôler et ajuster le pH, notamment par addition respective de d'une base ou d'un acide, en particulier la forme basique ou acide du composé formant tampon, et/ou contrôler et ajuster l'osmolarité, et
f) stériliser l'émulsion, en particulier par filtration de l'émulsion obtenue, notamment à l'aide d'un filtre de 0,22 µm, en particulier en la maintenant à une température supérieure à 30°C.

En particulier, l'agitation de l'étape d) est une agitation forte afin d'obtenir des gouttes de petites tailles, en particulier telles que définies dans la présente description. Cette agitation peut être effectuée avec un homogénéisateur.

Ledit procédé peut comprendre en outre une étape de conditionnement, en particulier dans un conditionnement stérile, et encore plus particulièrement dans un conditionnement permettant de conserver l'émulsion stérile, notamment le conditionnement est tel que décrit ci-dessus.

En particulier l'étape a) est effectuée en mélangeant le lipide autre que le phospholipide, le lipide de type phospholipide et de l'eau distillée, notamment en chauffant à une température supérieure à 30°C, sous agitation, jusqu'à obtention d'une phase homogène.

Le ratio de lipide de type phospholipide et de lipide autre que phospholipide peut aller de 1:5 à 1:1.

La teneur totale en lipide dans l'eau distillée peut être d'environ 0,5% en poids par rapport au poids total de la composition.

L'émulsion obtenue à l'étape a) peut être stérilisée par filtration, en particulier à l'aide d'un filtre de 0,22 µm. La température peut être supérieure à 30°C.

L'étape b) peut être effectuée en dissolvant le polymère mucomimétique dans un tampon, notamment citrate, et en chauffant, notamment à une température supérieure à 30°C.

Lors de l'étape c) il est possible de contrôler et ajuster le pH et/ou l'osmolarité par addition respective de citrate ou acide citrique et/ou de chlorure de sodium.

Lorsqu'énoncé que « la température peut être supérieure à 30°C », elle peut plus particulièrement aller de 32 à 45 °C, notamment aller de 35 à 43°C, voire aller de 37 à 42°C.

Tout particulièrement, cette émulsion est stérilisée par filtration, en particulier sur un filtre microbiologique de seuil de filtration de 0,22 µm, en PP (PolyPropylène), PES (PolyEtherSulfone), PTFE (PolyTétraFluoroEthylène), PET (PolyEster), PC (PolyCarbonate), ou tout autre filtre permettant d'effectuer une filtration stérilisante.

Selon une variante de mise en oeuvre du procédé, il est possible d'ajouter en outre durant l'étape a) à e) un surfactant, ou tensio-actif.

Selon une variante de mise en oeuvre du procédé, il est possible durant l'étape c) à e) d'ajouter un principe actif à usage ophtalmique, un agent conservateur et/ou un agent entrant dans la composition de la phase aqueuse du film lacrymal.

Bien entendu, les différentes caractéristiques exposées dans la présente description peuvent être combinées entre elles.

Les exemples suivants sont donnés à titre illustratif de l'invention.

### EXEMPLE

Les exemples ci-dessous sont des collyres à base d'émulsion H/E et d'un polymère mucomimétique, en particulier destinés à la restauration des différentes couches du film lacrymal chez les patients souffrant du syndrome de l'oeil sec. Ils sont préparés avec les ingrédients suivants donnés pour une composition centésimale. Ces formules présentent un pH d'environ 7,1 et sont hypotoniques aux larmes.

### Exemple comparatif 1 :

**Phase huileuse :**

| | |
|---|---|
| Triglycérides | 0,05% |
| Lécithine | 0,1% |

**Phase aqueuse :**

| | |
|---|---|
| Sulfate de chondroïtine | 0,2% |
| Alcool de Polyvinyle | 0,2% |
| Acide citrique | 0,05M |
| Citrate de sodium | 0,05M |
| Chlorure de sodium | q.s.p |
| Eau distillée | q.s.p. |

### Exemple comparatif 2 :

**Phase huileuse :**

| | |
|---|---|
| Triglycérides | 0,2% |
| Lécithine | 0,1% |

**Phase aqueuse :**

| | |
|---|---|
| Hyaluronate de sodium | 0,2% |
| Polysorbate | 0,2% |
| Acide citrique | 0,05M |
| Citrate de sodium | 0,05M |
| Chlorure de sodium | q.s.p |
| Eau distillée | q.s.p. |

### Exemple 3 :

**Phase huileuse :**

| | |
|---|---|
| Triglycérides | 0,05% |
| Lécithine | 0,1% |

**Phase aqueuse :**

| | |
|---|---|
| Sulfate de dextrane | 0,2% |
| Hydroxypropylméthylcellulose de sodium | 0,2% |
| Acide citrique | 0,05M |
| Citrate de sodium | 0,05M |
| Chlorure de sodium | q.s.p |
| Eau distillée | q.s.p. |

### Exemple comparatif 4:

**Phase huileuse :**

| | |
|---|---|
| Triglycérides | 0,05% |
| Lécithine | 0,1% |

**Phase aqueuse :**

| | |
|---|---|
| Héparine | 0,2% |
| Polyvinylpyrrolidone | 0,1% |
| Carboxyméthylcellulose de sodium | 0,1% |
| Acide citrique | 0,05M |
| Citrate de sodium | 0,05M |
| Chlorure de sodium | q.s.p |
| Eau distillée | q.s.p. |

### Exemple comparatif 5:

**Phase huileuse :**

| | |
|---|---|
| Triglycérides | 0,05% |
| Lécithine | 0,1% |

**Phase aqueuse :**

| | |
|---|---|
| Alginate de sodium | 0,1% |
| Alcool de Polyvinyle | 0,1% |
| Carboxyméthylcellulose de sodium | 0,2% |
| Acide citrique | 0,05M |
| Citrate de sodium | 0,05M |
| Chlorure de sodium | q.s.p |
| Eau distillée | q.s.p. |

### Exemple 6 :

**Phase huileuse :**

| | |
|---|---|
| Triglycérides | 0,1% |
| Lécithine | 0,1% |

**Phase aqueuse :**

| | |
|---|---|
| Hyaluronate de sodium | 0,2% |
| Carboxyméthylcellulose de sodium | 0,1% |
| Acide citrique | 0,05M |
| Citrate de sodium | 0,05M |
| Chlorure de sodium | q.s.p |
| Eau distillée | q.s.p. |

### Exemple 7 :

**Phase huileuse :**

| | |
|---|---|
| Triglycérides | 0,1% |
| Lécithine | 0,1% |

**Phase aqueuse :**

| | |
|---|---|
| Hyaluronate de sodium | 0,2% |
| Carboxyméthylcellulose de sodium | 0,05% |
| Alcool de polyvinyle | 0,1% |
| Acide citrique | 0,05M |
| Citrate de sodium | 0,05M |
| Chlorure de sodium | q.s.p |
| Eau distillée | q.s.p. |

### Exemple comparatif 8 :

**Phase huileuse :**

| | |
|---|---|
| Triglycérides | 0,05% |
| Lécithine | 0,1% |

**Phase aqueuse :**

| | |
|---|---|
| Hyaluronate de sodium | 0,2% |
| Alcool de polyvinyle | 0,2% |
| Acide citrique | 0,05M |
| Citrate de sodium | 0,05M |
| Chlorure de sodium | q.s.p |
| Eau distillée | q.s.p. |

### Exemple comparatif 9 :

**Phase huileuse :**

| | |
|---|---|
| Triglycérides | 0,2% |
| Lécithine | 0,1% |

**Phase aqueuse :**

| | |
|---|---|
| Hyaluronate de sodium | 0,2% |
| Polysorbate | 0,1% |
| Acide citrique | 0,05M |
| Citrate de sodium | 0,05M |
| Chlorure de sodium | q.s.p |
| Eau distillée | q.s.p. |

### Exemple 10 :

**Phase huileuse :**

| | |
|---|---|
| Triglycérides | 0,05% |
| Lécithine | 0,1% |

**Phase aqueuse :**

| | |
|---|---|
| Hyaluronate de sodium | 0,2% |
| Hydroxypropylméthylcellulose de sodium | 0,2% |
| Acide citrique | 0,05M |
| Citrate de sodium | 0,05M |
| Chlorure de sodium | q.s.p |
| Eau distillée | q.s.p. |

### Exemple 11 :

**Phase huileuse :**

| | |
|---|---|
| Triglycérides | 0,1% |
| Lécithine | 0,1% |

**Phase aqueuse :**

| | |
|---|---|
| Hyaluronate de sodium | 0,2% |
| Carboxyméthylcellulose de sodium | 0,1% |
| Hydroxypropylméthylcellulose de sodium | 0,1% |
| Acide citrique | 0,05M |
| Citrate de sodium | 0,05M |
| Chlorure de sodium | q.s.p |
| Eau distillée | q.s.p. |

### Exemple 12 :

**Phase huileuse :**

| | |
|---|---|
| Triglycérides | 0,05% |
| Lécithine | 0,1% |

**Phase aqueuse :**

| | |
|---|---|
| Hyaluronate de sodium | 0,2% |
| Carboxyméthylcellulose de sodium | 0,1% |
| Polyvinylpyrrolidone | 0,1% |
| Acide citrique | 0,05M |
| Citrate de sodium | 0,05M |
| Chlorure de sodium | q.s.p |
| Eau distillée | q.s.p. |

### Exemple 13 :

**Phase huileuse :**

| | |
|---|---|
| Triglycérides | 0,05% |
| Lécithine | 0,1% |

**Phase aqueuse :**

| | |
|---|---|
| Hyaluronate de sodium | 0,2% |
| Carboxyméthylcellulose de sodium | 0,1% |
| Alcool de Polyvinyle | 0,1% |
| Acide citrique | 0,05M |
| Citrate de sodium | 0,05M |
| Chlorure de sodium | q.s.p |
| Eau distillée | q.s.p. |

## Revendications

1. Emulsion de type huile dans eau dépourvue de conservateurs, dont les globules présentent une taille maximale inférieure ou égale à 220 nm, ayant une osmolarité allant de 110 et 180 mOsm/l, comprenant :
- au moins un polymère mucomimétique choisi parmi les acides hyaluroniques, le sulfate de dextrane et le sulfate de chondroïtine,
- au moins un lipide de type phospholipide choisi parmi les lécithines,
- au moins un lipide autre que le phospholipide,
- au moins un polymère stabilisant choisi parmi les polymères de cellulose,
- milieu tampon citrate,
- de l'eau,
pour son utilisation dans le traitement ou la prévention des affections ophtalmiques.

2. Emulsion de type huile dans eau pour son utilisation selon la revendication 1 comprenant en outre un agent thérapeutique à destination ophtalmique.

3. Emulsion pour son utilisation selon la revendication 1 ou 2 pour son utilisation dans le traitement ou la prévention de la sécheresse oculaire, les allergies et/ou les inflammations au niveau de l'oeil.

4. Emulsion pour son utilisation selon l'une des revendications précédentes dans laquelle la teneur en polymère mucomimétique varie de 0,01 à 5 % en poids par rapport au poids total de l'émulsion.

5. Emulsion pour son utilisation selon l'une quelconque des revendications 1 à 4, dans laquelle la teneur en phospholipide varie de 0,01 à 3 % en poids par rapport au poids total de l'émulsion.

6. Emulsion pour son utilisation selon l'une quelconque des revendications 1 à 5 dans la laquelle la teneur en lipide autre que phospholipide varie de 0,01 à 5 % en poids par rapport au poids total de l'émulsion.

7. Emulsion pour son utilisation selon l'une des revendications 1 à 6 dans laquelle le polymère de cellulose est choisi parmi la méthylcellulose, l'éthylcellulose, l'hydroxypropylcellulose et la carboxyméthylcellulose.

8. Emulsion pour son utilisation selon l'une quelconque des revendications 2 à 7 dans laquelle la teneur en polymère stabilisant varie de 0,01 à 5 % en poids par rapport au poids total de l'émulsion.

9. Emulsion pour son utilisation selon l'une quelconque des revendications 1 à 8, **caractérisée en ce qu'**elle présente une viscosité inférieure ou égale à 10⁻¹ Pa.s.

10. Emulsion pour son utilisation selon l'une quelconque des revendications 1 à 9, **caractérisée en ce qu'**elle présente une limpidité allant de 0 à 5000 NTU.

11. Emulsion pour son utilisation selon l'une quelconque des revendications 2 à 8 présentant un pH allant de 5,5 à 8.

12. Procédé de préparation d'une émulsion huile dans eau telle que définie selon l'une quelconque des revendications 1 à 11, comprenant les étapes suivantes :
a) mélanger un lipide de type phospholipide choisi parmi les lécithines et un lipide autre que le phospholipide,
b) dissoudre un polymère mucomimétique choisi parmi les acides hyaluroniques, le sulfate de dextrane et le sulfate de chondroïtine dans un tampon citrate
c) dissoudre un polymère stabilisant choisi parmi les polymères de cellulose dans la phase aqueuse de l'étape b),
d) disperser la phase huileuse de l'étape a) dans la phase aqueuse de l'étape c) par agitation,
e) contrôler et ajuster le pH, notamment par addition respective d'une base ou d'un acide et/ou contrôler et ajuster l'osmolarité, et
f) stériliser l'émulsion, de préférence par filtration à l'aide d'un filtre de 0,22 µm.

## Patentansprüche

1. Öl-in-Wasser emulsion ohne Konservierungsstoffe, deren Kügelchen eine maximale Größe kleiner oder gleich 220 nm aufweisen, mit einer Osmolarität von 110 bis 180 mOsm/l, umfassend:
- mindestens ein mucomimetisches Polymer, ausgewählt aus den Hyaluronsäuren, dem Dextransulfat und dem Chondroitinsulfat,
- mindestens ein Lipid vom Typ Phospholipid, ausgewählt aus den Lecithinen,
- mindestens ein Lipid, das kein Phospholipid ist,
- mindestens ein stabilisierendes Polymer, ausgewählt aus den Cellulosepolymeren,
- Citratpuffermedium,
- Wasser
zur Verwendung bei der Behandlung oder Vorbeugung von Augenerkrankungen.

2. Öl-in-Wasser emulsion zur Verwendung nach Anspruch 1, umfassend ferner ein therapeutisches Mittel für die Augen.

3. Emulsion zur Verwendung nach Anspruch 1 oder 2 für ihre Verwendung bei der Behandlung oder Vorbeugung von Augentrockenheit, Allergien und/oder Augenentzündungen.

4. Emulsion zur Verwendung nach einem der vorangehenden Ansprüche, wobei der Gehalt an mucomimetischem Polymer von 0,01 bis 5 Gew.-% in Bezug zum Gesamtgewicht der Emulsion schwankt.

5. Emulsion zur Verwendung nach einem der Ansprüche 1 bis 4, wobei der Gehalt an Phospholipid von 0,01 bis 3 Gew.-% in Bezug zum Gesamtgewicht der Emulsion schwankt.

6. Emulsion zur Verwendung nach einem der Ansprüche 1 bis 5, wobei der Gehalt an Lipid, das kein Phospholipid ist, von 0,01 bis 5 Gew.-% in Bezug zum Gesamtgewicht der Emulsion schwankt.

7. Emulsion zur Verwendung nach einem der Ansprüche 1 bis 6, wobei das Cellulosepolymer aus der Methylcellulose, der Ethylcellulose, der Hydroxypropylcellulose und der Carboxymethylcellulose ausgewählt ist.

8. Emulsion zur Verwendung nach einem der Ansprüche 2 bis 7, wobei der Gehalt an stabilisierendem Polymer von 0,01 bis 5 Gew.-% in Bezug zum Gesamtgewicht der Emulsion schwankt.

9. Emulsion zur Verwendung nach einem der Ansprüche 1 bis 8, **dadurch gekennzeichnet, dass** sie eine Viskosität kleiner oder gleich 10⁻¹ Pa.s aufweist.

10. Emulsion zur Verwendung nach einem der Ansprüche 1 bis 9, **dadurch gekennzeichnet, dass** sie eine Klarheit von 0 bis 5000 NTU aufweist.

11. Emulsion zur Verwendung nach einem der Ansprüche 2 bis 8, aufweisend einen pH von 5,5 bis 8.

12. Herstellungsverfahren einer Öl-in-Wasser-Emulsion nach einem der Ansprüche 1 bis 11, umfassend die folgenden Schritte:
a) Mischen eines Lipids vom Typ Phospholipid, ausgewählt aus den Lecithinen, und ein Lipid, das kein Phospholipid ist,
b) Auflösen eines mucomimetischen Polymers, ausgewählt aus den Hyaluronsäuren, dem Dextransulfat und dem Chondroitinsulfat, in einem Citratpuffer,
c) Auflösen eines stabilisierenden Polymers, ausgewählt aus den Cellulosepolymeren, in der wässrigen Phase von Schritt b),
d) Dispergieren der öligen Phase von Schritt a) in der wässrigen Phase von Schritt c) durch Rühren,
e) Kontrollieren und Einstellen des pH, vor allem durch jeweiliges Hinzufügen einer Base oder einer Säure, und/oder Kontrollieren und Einstellen der Osmolarität, und
f) Sterilisieren, vorzugsweise durch Filtration mit Hilfe eines Filters von 0,22 µm.

## Claims

1. A preservative-free oil-in-water emulsion, the globules of which have a maximum size of 220 nm or less, having an osmolarity of 110 to 180 mOsm/L, comprising:
- at least one mucomimetic polymer selected from hyaluronic acids, dextran sulfate and chondroitin sulfate,
- at least one lipid of the phospholipid type selected from lecithins,
- at least one lipid other than the phospholipid,
- at least one stabilizing polymer selected from cellulose polymers,
- citrate buffer medium,
- water,
for use in treating or preventing ophthalmic diseases.

2. The oil-in-water emulsion for use according to claim 1 further comprising a therapeutic agent for ophthalmic use.

3. The emulsion for use according to claim 1 or 2 for use in treating or preventing ocular dryness, allergies and/or inflammation of the eye.

4. The emulsion for use according to one of the preceding claims wherein the mucomimetic polymer content varies from 0.01 % to 5 % by weight in relation to the total weight of the emulsion.

5. The emulsion for use according to any one of claims 1 to 4, wherein the phospholipid content varies from 0.01 % to 3 % by weight in relation to the total weight of the emulsion.

6. The emulsion for use according to any one of claims 1 to 5 wherein the content of lipid other than the phospholipid varies from 0.01 % to 5 % by weight in relation to the total weight of the emulsion.

7. The emulsion for use according to one of claims 1 to 6 wherein the cellulose polymer is selected from methylcellulose, ethylcellulose, hydroxypropylcellulose and carboxymethylcellulose.

8. The emulsion for use according to any one of claims 2 to 7 wherein the stabilizing polymer content varies from 0.01 % to 5 % by weight in relation to the total weight of the emulsion.

9. The emulsion for use according to any one of claims 1 to 8, **characterized in that** said emulsion has a viscosity of 10⁻¹ Pa.s or less.

10. The emulsion for use according to any one of claims 1 to 9, **characterized in that** said emulsion has a clarity of 0 to 5000 NTU.

11. The emulsion for use according to any one of claims 2 to 8 having a pH of 5.5 to 8.

12. A method for preparing an oil-in-water emulsion as defined according to any one of claims 1 to 11, comprising the following steps:
a) mix a lipid of the phospholipid type selected from lecithins and a lipid other than the phospholipid,
b) dissolve a mucomimetic polymer selected from hyaluronic acids, dextran sulfate and chondroitin sulfate in citrate buffer,
c) dissolve a stabilizing polymer selected from cellulose polymers in the aqueous phase of step b),
d) disperse the oily phase of step a) in the aqueous phase of step c) by agitation,
e) control and adjust the pH, notably by adding respectively a base or an acid and/or control and adjust the osmolarity, and
f) sterilize the emulsion, preferably by filtration using a 0.22 µm filter.
